# EUROPEAN PATENT APPLICATION

(11) **EP 0 698 398 A1**
(43) Date of publication of application: **28.02.1996**
(21) Application number: 94113106.2
(22) Date of filing: 23.08.1994
(51) Int. Cl.: A61L 33/00

(54) **Blood collection device**

(71) Applicant: Becton Dickinson and Company, Franklin Lakes, New Jersey 07417-1880 (US)
(72) Inventor: Dufresne, Christopher M., F-38560 Jarrie (GB); Vigier, Jean-Pierre, M.D., F-38610 Gieres (GB); Dowell, David, Landrake PL 125DN (GB)
(74) Representative: von Kreisler, Alek, Dipl.-Chem.

(57) **Abstract**

A blood collection device comprising an anticoagulant formulation on the interior surface of the device. The anticoagulant formulation on the interior surface of the device is an improvement over available devices that contain liquid anticoagulant formulations. The device is coated by combining sodium citrate and citric acid in a water vehicle, spraying the formulation onto the interior surface of the device, followed by air drying.

## Description

### BACKGROUND OF THE INVENTION

### 1. Field of the invention

This invention relates to a blood collection device for use in coagulation and hematology studies. More particularly, the present invention pertains to a blood collection device comprising an anticoagulant formulation and the method for imparting the anticoagulant formulation to the device.

### 2. Description of Related Art

As more medical devices made of plastic materials are used frequently and diversly, there has been a demand for improved anticoagulant formulation fill into the devices. Such improvement is conducive to collection tubes, especially blood collection tubes among plastic medical devices. Blood collection tubes need to be designed in such a manner that anticoagulant formulations in tubes efficiently work in tests or analysis, and the materials of the tubes do not interfere with testing or analysis. Such tests include but are not limited to hematology, blood chemistry, blood typing, toxicology analysis and therapeutic drug monitoring.

Freeze drying, vacuum drying and liquid filling are the conventional methods for filling anticoagulant formulations into blood collection devices. However, these conventional methods have the following disadvantages. In the case of freeze-drying, in plastic blood collection devices, anticoagulant formulations can be rehydrated again after drying due to moisture permeation into the plastic device, which is not desirable. With drying methods, the anticoagulant formulations tend to be localized within the tube. In addition, liquid filling is not practical because certain liquid anticoagulant formulations will degrade with irradiation during sterilization or due to the moisture permeation out of a plastic device.

### SUMMARY OF THE INVENTION

The present invention is a blood collection device with an anticoagulant formulation spray dried to the interior wall of the device.

Preferably, the anticoagulant formulation comprises sodium citrate (C₆H₅O₇Na₃2H₂O) and citric acid (HOOCCH₂C(OH)(COOH)CH₂COOH-H₂O).

Most preferably, the anticoagulant formulation comprises:
(a) from about 2.7 to about 3.6 (mgs) of tri-sodium citrate per ml of blood; and
(b) from about 0.42 to about 0.50 (mgs) of citric acid per ml of blood.

The method for coating substrates with an anticoagulant formulation comprises:
(a) preparing an anticoagulant formulation comprising admixing:
   (i) a liquid vehicle for the formulation;
   (ii) sodium citrate; and
   (iii) citric acid.
(b) selecting a substrate to be coated;
(c) applying the anticoagulant formulation to the interior surface of the substrate with a means that produces a fine mist of the formulation; and
(d) drying the applied formulation by applying an air jet or forced air to the coated substrate at an elevated temperature for a period of time.

Preferably, the anticoagulant formulation of the present invention is spray dried with a water carrier onto the interior wall of a blood collection device. Most preferably, the blood collection device is a blood collection tube made of glass or plastic. A subsequent drying step which may be simple air drying achieves the resulting dried coating.

With this invention, by contrast to the commercially available liquid additive tubes, it has now been found that an anticoagulant formulation comprising tri-sodium citrate and citric acid can be imparted to a blood collection tube, which resulting coating will provide the desired anticoagulant properties to the substrate being coated.

Blood collection tubes spray dried with the anticoagulant formulation of the present invention is an improvement over commercially available blood collection tubes that consist of liquid anticoagulant formulations. Such commercially available products include VACUTAINER Brand glass tubes containing a liquid additive of 0.500 mls of 0.129 Molar Na₃ Citr/Citr. Acid (Becton, Dickinson and Company, 1 Becton Drive, Franlin Lakes, NJ 07417). However, the liquid anticoagulant in glass tubes is not useful in plastic blood collection tubes. Plastic blood collection tubes do not have sufficient vapor and water properties to preserve such a liquid anticoagulant.

Spraying the anticoagulant formulation over a large surface area substantially reduces the local osmolarity and concentration gradients between the citrate and the blood cells thereby minimizing the possibility of cell rupturing and hemolysis. This may occur if a powder additive or freeze dried additive is used. The anticoagulant formulation of the present invention also provides better contact with the blood specimen and therefore rapid dissolution of the anticoagulant formulation into the blood specimen is facilitated and prevents the initiation of blood clotting mechanisms.

When performing coagulation studies, it is important that the 9 parts blood to 1 part liquid citrate additive solution be maintained as certain coagulation parameters or times may be lengthened. In the case of a glass tube, moisture loss is not present to any appreciable amount thus, the amount of water and the Na₃/citric acid remain constant for long periods. When blood is drawn into the evacuated tube to the proper amount, the 9:1 ratio of blood to additive is maintained.

For plastic tubes, there is significant moisture loss unless special barrier containers and packaging are employed. Without the special barrier containers and packaging or once the plastic tube is removed from the package, there is rapid moisture loss. The Na₃/citric acid remains in the tube but the moisture evaporates. The result is the remaining solution increases in molarity as more and more water evaporates. Although there is sufficient Na₃/citric acid to anticoagulate the blood because the Na₃/citric acid does not permeate the tube, the 9:1 ratio with the water is gone. Eventually, all the water is gone and the Na₃/citric acid is left as a solid somewhere in the tube. Therefore, a single mass or pellet may exist. The surface area of this dried mass would be small and solubility of the mass would be very poor when the blood enters the tube. The outcome is hemolysis of blood and microclotting of the blood making coagulation studies impossible. In addition, because the blood to water ratio is unpredictable over the days and weeks immediately after the plastic tube is removed from its package, a significant error could be introduced into the measurement of the coagulation parameters.

In the case of the present invention, a plastic tube with spray dried citrate, there is no moisture loss to be concerned about and no special packages are needed. Sufficient citrate is sprayed over a large area of the tube to achieve the anticoagulation properties needed without hemolysis and with rapid solubility. Coagulation parameters can be established for the product and because there is no loss of moisture, the repeatability of these parameters is greatly improved in comparison to plastic tubes containing liquid citrate solutions.

The spray dried anticoagulant formulation of the present invention has the further advantage over a liquid anticoagulant formulation in that there is no dilution of the blood specimen and therefore no dilution of the concentration of the analytes that may be examined. When performing coagulation studies (prothrombin times, thrombin time, anti-prothrombin time, etc.), typical citrate anticoagulant formulations have a 9:1 blood to additive volume ratio which is believed to lengthen certain coagulation parameters or times. When performing erythrocyte sedimentation rate (ESR) measurement (for example, westergren determination), citrate anticoagulant formulations have a 4:1 blood to additive volume ratio which substantially decreases the rate of settling of cells such as leukocytes, thrombocytes and erythrocytes during the measurement of the ESR.

In the case of tubes used for ESR measurements, the same as above applies as well. In this instance, the blood additive ratio recommended for performing a classic WESTERGEN Determination is 4:1. Again for glass tubes, moisture loss and conservation of this ratio is not a problem but plastic tubes require special packages. In the case of plastic tubes, if the amount of liquid present is unpredictable or not constant, the measurement of the ESR will vary. In tubes with the specified amount of liquid at a 4:1 ratio, the cellular components of the blood are diluted and this tends to make them descend faster than an undiluted specimen. If significant moisture has been lost, the cells will fall more slowly giving a different ESR value.

The advantage provided by the present invention, is that citrate is sprayed over a large surface of the tube and there is no liquid to worry about. Having established an appropriate correlation of the tube containing spray dried citrate to the Westergren Value, the moisture loss is no longer significant in a plastic tube intended for ESR determinations.

Most notably, the spray dried anticoagulant formulation of the present invention provides a stable blood to additive/concentration over the shelf life of a blood collection device. Currently, plastic tubes containing liquid anticoagulant formulations are packaged in special barrier packages that minimize the rate of loss of the water component of the liquid formulation from the tube. Without such packaging, the rate of water loss is so rapid that within time the liquid formulation has permeated the tube wall and the liquid formulation and blood concentration is then adversely impacted, thus changing the coagulation and hematology results. Furthermore, there is a cost advantage with the device and the method for making the same according to the present invention. In the case of a plastic tube containing the formulation, no special barrier packages are required to prevent moisture loss. Thus, cost reductions in manufacturing due to the elimination of unnecessary packaging material is substantially achieved.

The anticoagulant dissolution in a blood collection tube spray dried with an anticoagulant formulation is improved by a number of factors. In the case of a plastic blood collection tube, the plastic material used for the tube has a lower surface energy than that of glass. The lower surface energy results in the anticoagulant remaining on the tube wall after the spray coat application rather than running down the tube wall and gathering in the bottom of the tube. With the anticoagulant remaining where applied inside the tube, the anticoagulant can be applied along the entire internal surface area of the tube. This makes the anticoagulant available to more of the specimen and decreases the anticoagulant dissolution time into the specimen. Also, by making the anticoagulant available to more of the specimen, risk of specimen microclots is decreased.

Thus the method and formulation of the present invention imparts to the internal surfaces of devices, such as plastic blood collection devices, anticoagulant properties equivalent when compared to glass blood collection device with liquid citrate and improved when compared to tubes containing powdered or freeze dried or vacuum dried citrate. The method and formulation of the present invention is also an improvement over plastic tubes containing liquid citrate solutions.

### DESCRIPTION OF THE DRAWINGS

FIG. 1 is a perspective view of a typical blood collection tube with a stopper.

FIG. 2 is a longitudinal sectional view of the tube of FIG. 1, taken along line 2-2, comprising a spray dried anticoagulant formulation of the present invention.

### DETAILED DESCRIPTION

The present invention may be embodied in other specific forms and is not limited to any specific embodiments described in detail which are merely exemplary. Various other modifications will be apparent to and readily made by those skilled in the art without departing from the scope and spirit of the invention. The scope of the invention will be measured by the appended claims and their equivalents.

The blood collection devices of the present invention preferably comprises an anticoagulation formulation comprising sodium citrate and citric acid.

Most preferably, the anticoagulant formulation comprises:
(a) from about 2.7 to about 3.6 mgs of tri-sodium citrate per ml of blood; and
(b) about 0.42 to about 0.50 mgs of citric acid per ml of blood.

The anticoagulant formulation is applied onto an interior wall of a tube in the form of fine mist by a spray coating process using a carrier solvent, preferably water. The main advantages of a tube with a spray coated anticoagulant formulation on the tube interior wall are more precise, stable, uniform and dry anticoagulant fill and improved anticoagulant dissolution into blood specimen. Because of the fine mist of the anticoagulant formulation, the actual surface area of anticoagulant formulation exposed to blood is maximized.

It is preferable that the anticoagulant formulation is metered and dispensed by a volumetric type device, such as a positive displacement pump. The solution concentration (amount of anticoagulant per unit volume of formulation) is tailored with the dispense volume so that the desired amount of anticoagulant is dispensed into the device. Other spraying techniques include ultrasonic spraying.

Most preferably, the applied formulation is dried by applying an air jet to the coated substrate.

A blood collection device of the present invention can be either an evacuated blood collection device or a non-evacuated blood collection device. The blood collection device is desirably made of polyethylene terephthalate, polypropylene or glass.

Tri-sodium citrate and citric acid are very soluble in blood and water. These components are relatively easy to formulate, mix and dispense and are effective in rapidly anticoagulating blood.

Water can be used as the solvent without other volatile solvents such as freon. Water has an advantage in that it has minimal detrimental effects on product or environment.

A variety of tube coatings or the addition of other compounds to the anticoagulant formulation may be desirable. Such things include but are not limited to, silicone oils and silicone surfactants.

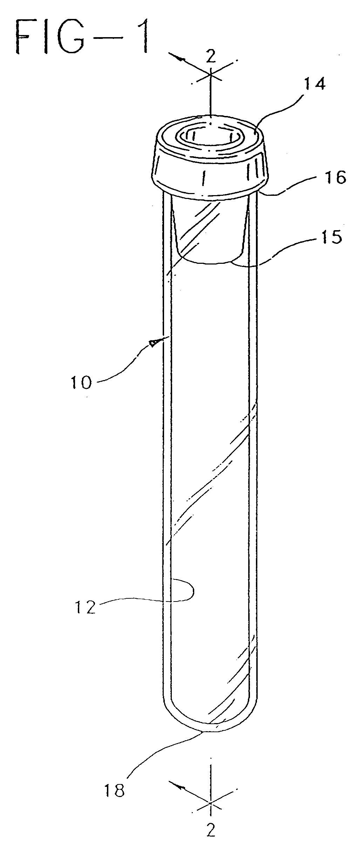

Referring to the drawings in which like reference characters refer to like parts throughout the several views thereof, FIG. 1 shows a typical blood collection tube **10**, having an open end **16**, a closed end **18** and a stopper **14** that includes a lower annular portion or skirt **15** which extends into and presses against the inside wall 12 of the tube for maintaining stopper **14** in place.

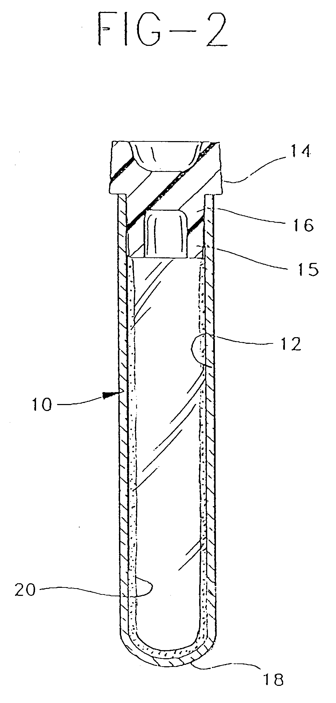

FIG. 2 shows the anticoagulant formulation of the present invention in a typical blood collection tube. An anticoagulant formulation **20** is shown adjacent inside wall **12** of the tube.

A blood sample of interest can be transferred into tube **10** that comprises anticoagulant formulation **20**. The blood sample efficiently contacts anticoagulant formulation **20** so that anticoagulant formulation **20** rapidly dissolves into the blood sample and clotting of the blood sample is prohibited.

Various other modifications will be apparent to and may be readily made by those skilled in the art without departing from the scope and spirit of the invention.

The examples are not limited to any specific embodiment of the invention but are only exemplary.

### EXAMPLE I

### METHOD OF COATING TUBES WITH AN ANTICOAGULANT FORMULATION

An anticoagulant formulation was prepared comprising about 426.7g of tri-sodium citrate and 56.7g of citric acid dissolved in 1000 mls of distilled water. The obtained formulation was sprayed onto the interior walls of 1000 test tubes molded from polyethylene terepthalate plastic (having an internal volume of 4.0ml) using a spray coating process. In each tube, 0.030 ml of the formulation was sprayed. The formulation was also sprayed into 1,000 glass tubes (having an internal volume of 4.5 ml). In each glass tube, 0.034 ml of the formulation was sprayed.

The anticoagulant formulation was then dried by forced hot air. The test tubes were each evacuated and sealed with a closure and sterilized by gamma irradiation at 2.5 mega Rads.

Product was evaluated and shown to be functional. Specifically, rapid solubility of the anticoagulant formulation was demonstrated when deionized water was drawn into the test tubes and the tubes were inverted 5 times. The closure was removed and the water poured out. The interior tube wall was examined and found to be free of the spray dried anticoagulant formulation. Subsequently, another set of test tubes were used to collect blood specimens using standard venipuncture techniques. The tubes were inverted 5 times. The tubes were examined for the formation of clots and were found to be clot free. The tubes were then centrifuged and the resulting plasma was visually examined for hemolysis and was found to be hemolysis free. The tubes were refrigerated at 4°C for 40 hours. They were remixed to suspend the cells, re-centrifuged and re-examined for clots and hemolysis and found to be free from clots or hemolysis.

All of the above tests were performed in parallel using a glass tube containing 0.5 mls of liquid citrate solutions of 0.105 and 0.129 Molar Na₃ citrate/citric acid. Solubility with 5 inversions of the liquid solution with water or blood specimens was immediate and complete. The results indicated that no clots had formed and that the resulting plasma was hemolysis free.

In conclusion, it has been shown that the spray dried Na₃/citric acid formulations of the present invention or glass or plastic tubes provide a highly soluble and effective method of anti-coagulating the specimen without hemolysis in comparison to a glass tube containing liquid Na₃/citric acid solutions.

## Claims

1. A blood collection device comprising a top end, a bottom end, a sidewall extending from said top end to said bottom end and including an exterior and interior surface, and a spray coated anticoagulant formulation comprising tri-sodium, citrate and citric acid, on said interior surface applied by a spray coating process which employs water as a carrier solvent which is subsequently substantially removed leaving a dry additive.

2. The device of Claim 1, wherein said blood collection device is made of polyethylene terephthalate, polypropylene or glass.

3. The device of Claim 1, wherein the concentration of tri-sodium citrate is about 2.7 mgs to about 3.6 mgs per ml of blood and the concentration of citric acid is about 0.42 mgs to about 0.50 mgs per ml of blood.

4. A method for coating substrates comprising the steps of:
(a) preparing an anticoagulant formulation of tri-sodium citrate and citric acid and water;
(b) selecting a substrate to be coated;
(c) dispersing said formulation to said substrate in a fine mist; and
(d) drying said applied formulation by applying forced air to the coated substrate for a sufficient period of time to dry the formulation whereby a dry formulation remains.

5. The method of Claim 4, wherein said substrate is a plastic or glass material.

6. The method of Claim 5, wherein the said substrate is a blood collection device.

7. The method of Claim 6, wherein the concentration of tri-sodium citrate is about 2.7 mgs to about 3.6 mgs per ml of blood and citric acid is about 0.42 mgs to about 0.50 mgs per ml of blood.

8. The device of Claim 1, wherein said spray coating process comprises:
(a) preparing a coating solution of said anticoagulant formulation and water;
(b) dispersing said coating solution to said device in a fine mist; and
(c) drying said applied coating by applying forced air to the coated device for sufficient period of time to dry the coating whereby a dry formulation remains.
